# EUROPÄISCHE PATENTSCHRIFT

(11) **EP 2 114 308 B1**
(45) Veröffentlichungstag und Bekanntmachung des Hinweises auf die Patenterteilung: **14.03.2018**
(21) Anmeldenummer: 08700862.9
(22) Anmeldetag: 16.01.2008
(51) Int. Cl.: A61F 2/30, A61F 2/44

(54) **ZWISCHENWIRBELIMPLANTAT MIT ELASTISCHEM BAUTEIL**
INTERVERTEBRAL IMPLANT HAVING AN ELASTIC COMPONENT
IMPLANT INTERVERTÉBRAL COMPORTANT UN COMPOSANT ÉLASTIQUE

(30) Priorität: 07.03.2007 DE 102007011059
(43) Veröffentlichungstag der Anmeldung: 11.11.2009
(73) Patentinhaber: Ulrich GmbH & Co. KG, 89081 Ulm (DE)
(72) Erfinder: RICHTER, Marcus, 65191 Wiesbaden (DE); WILLMANN, Nicolas, 89077 Ulm (DE); HAMICH, Sven, 89075 Ulm (DE)
(74) Vertreter: Hentrich Patentanwälte PartG mbB
(86) Internationale Anmeldenummer: PCT/DE2008/000072
(87) Internationale Veröffentlichungsnummer: WO 2008/106912

(56) Entgegenhaltungen:
- EP-A- 0 538 183
- EP-A- 1 398 008
- WO-A-98/22050
- WO-A-2004/043304
- WO-A-2005/039455
- DE-U1- 20 019 520
- US-A- 6 136 031
- US-A1- 2001 051 829
- US-A1- 2004 220 671
- US-A1- 2005 113 924
- US-A1- 2005 187 627

## Beschreibung

Die Erfindung betrifft ein Implantat zum Einsetzen zwischen Wirbelkörper der Wirbelsäule, das eine erste Kontaktfläche zur Anlage an einen der benachbarten Wirbelkörper und eine zweite Kontaktfläche zur Anlage an den anderen benachbarten Wirbelkörper aufweist und eine Kraftübertragungskette zwischen den Anlageflächen der benachbarten Wirbelkörper bildet, in der mindestens ein elastisch nachgiebiges Bauteil angeordnet ist.

Implantate sind beispielsweise aus der DE 44 23 257 A1 bekannt, die einen möglichen grundsätzlichen Aufbau des erfindungsgemäßen Implantates zeigt. Gleiches gilt auch für die EP 1 721 583 A2, die ein Implantat offenbart, dessen Aufbau als Ausgangspunkt für die Erfindung geeignet ist.

Ein Implantat der eingangs genannten Art ist aus der WO 2005/039455 bekannt, bei dem das Implantatteil als unten geschlossener Topf gebildet ist. Dokument US 2005/0113924 A1 offenbart die im Oberbegriff des Anspruchs 1 genannte Merkmale. Bei bekannten, zwischen die Wirbelkörper der Wirbelsäule einzusetzenden Implantaten besteht bei den bisher aus dem Stand der Technik bekannten Ausführungsformen das Problem, dass es zu einer Überlastung der Wirbelkörperendplatten kommen kann mit dem Ergebnis, dass dieser einbrechen kann und dadurch auch die dem Implantat benachbarten Wirbelkörper beschädigt oder gar zerstört werden.

Der Erfindung liegt daher die Aufgabe zugrunde, ein Implantat der eingangs genannten Art so auszubilden, dass die Belastung der Wirbelkörperendplatten reduziert wird.

Diese Aufgabe wird nach der Erfindung bei einem Implantat der eingangs genannten Art dadurch gelöst, dass das Implantat mehrteilig mit mindestens einem ersten Implantatteil und einem zweiten Implantatteil gebildet ist, die in Richtung ihrer Längsachse relativ zueinander verstellbar sind, dass jedes der Implantatteile als rohrförmige Hülse gebildet ist, und dass mindestens eines der Implantatteile das in der Wandung dieses Implantatteils integrierte elastische nachgiebige Bauteil aufweist, das durch Schlitze in einer kreuzweise versetzten Anordnung gebildet ist.

Durch diese Gestaltung ergibt sich der Vorteil, dass eine gleichmässige Verteilung der Last auf die Anlagefläche der Wirbelkörperendplatten der benachbarten Wirbelkörper erreicht wird, da durch das elastisch nachgiebige Bauteil eine Anpassung des Implantats an die Orientierung der Anlageflächen ermöglicht ist. Die Kontaktfläche ist dadurch vergrößert und insbesondere eine einseitige Linienberührung vermieden, so dass es nicht lokal zu einem Einbrechen der Wirbelkörperendplatten kommen kann mit einem sich nachfolgend ausbreitenden Bruch. Bei dieser Gestaltung muss das elastisch nachgiebige Bauteil nicht endständig beim Implantatteil positioniert werden, was natürlich ergänzend gleichfalls möglich ist, sondern kann die endständigen Kontaktflächen frei geben und die gewünschte Elastizität des Implantats in einer beliebiger Höhenlage seiner Längsstreckung bereitstellen. Die verbesserte Anlage der Implantatteile an den benachbarten Wirbelkörpern schließt dabei auch eine Konfiguration ein, bei der das Implantatteil in dem elastisch nachgiebigen Bauteil abgeknickt ist, also im Prinzip ein Gelenk bereitgestellt ist, das darüber hinaus auch die Funktion der Bandscheiben mit übernimmt und Belastungsspritzen bzw. Stöße abfedert. Die Verstellbarkeit in Richtung ihrer Längsachse schafft eine Distraktionsmöglichkeit, also das Implantat in einer kurzen Konfiguration einfach in die zwischen den beiden Wirbelkörpern bestehenden Lücke eingeführt werden kann, um nachfolgend im Laufe der Operation auf die erforderliche Länge distrahiert zu werden.

Im Rahmen der Erfindung ist es bevorzugt, wenn das mindestens eine elastisch nachgiebige Bauteil der ersten Kontaktflächen und/oder der zweiten Kontaktfläche zugeordnet ist, da so eine gleichmäßige Kraftverteilung an den Implantatteilen endständig realisiert wird, also prinzipiell bereits bekannte Implantatteile durch die erfindungsgemäßen Merkmale ergänzt und weitergebildet werden können, ohne in deren grundsätzlichen Aufbau eingreifen zu müssen.

Es bietet sich an, dass in einer Radialebene der Hülse zwei diametral gegenüberliegende Schlitze ausgebildet sind, die gegenüber den Schlitzen in den benachbarten Radialebenen um 90° um die Längsachse der Hülse gedreht angeordnet sind, so dass durch das Einbringen der Schlitze in die Wandung der Hülse nachträglich die gewünschte Elastizität hergestellt werden kann.

Um eine individuelle Anpassung des Implantats an die Patienten zu ermöglichen, ist vorgesehen, dass der Hülse endständige Ansatzplatten zugeordnet sind, die die erste Kontaktfläche und die zweite Kontaktfläche bilden. Dann besteht auch die Möglichkeit, dass die Ansatzplatten die elastisch nachgiebigen Bauteile bilden, so dass durch geeignete Wahl der Bauform und Materialeigenschaften der Ansatzplatten wiederum eine individuelle Anpassung ermöglicht ist.

Da die Ansatzplatten separat von der Hülse gefertigt werden können, besteht auch die Möglichkeit, dieses in Längsrichtung der Hülse weisenden Dichtegradienten zuzuordnen, um so eine Elastizität zu erreichen.

Eine weitere alternative Ausführungsform ist dadurch gekennzeichnet, dass die Ansatzplatten zur Anlage an die benachbarten Wirbelkörper bestimmte Stifte tragen, die in einem flexiblen Poster gelagert sind, so dass auf diese Weise eine gleichmäßige Anlage an die Wirbelkörperendplatten ermöglicht ist.

Eine Berücksichtigung der natürlichen Krümmung der Wirbelsäule wird ermöglicht, indem die Endplatten gelenkig an der Hülse gelagert sind.

Die Verstellbarkeit in Richtung der Längsachse lässt sich dabei in besonders einfacher Weise erzielen, indem das erste Implantatteil und das zweite Implantatteil durch ein Distraktionsgewinde miteinander verbunden sind, wobei wiederum zur Erweiterung der konkreten Realisierung des Distraktionsgewindes auf die eingangs genannten Druckschriften derselben Anmelderin verwiesen werden können.

Im folgenden wird die Erfindung an in der Zeichnung dargestellten Ausführungsbeispielen näher erläutert; es zeigen:
Fig. 1 eine Seitenansicht eines Implantats, teilweise geschnitten dargestellt mit einem in der Wandung der Hülse in einer Wabenstruktur ausgebildeten elastischen nachgiebigen Bauteil,
Fig. 2 eine der Figur 1 entsprechende Darstellung einer Ausführungsform mit einem in die Wandung der Hülse integrierten elastisch nachgiebigen Bauteil aus einem elastisch kompakten Material,
dies bei einem einteiligen Implantat allerdings aufwändig in der Herstellung ist, so dass sich dann eine mehrteilige Ausgestaltung anbietet.

Um eine individuelle Anpassung des Implantats an die Patienten zu ermögichen, ist vorgesehen, dass der Hülse endständige Ansatzplatten zugeordnet sind, die die erste Kontaktfläche und die zweite Kontaktfläche bilden. Dann besteht auch die Möglichkeit, dass die Ansatzplatten die elastisch nachgiebigen Bauteile bilden, so dass durch geeignete Wahl der Bauform und Materialeigenschaften der Ansatzplatten wiederum eine individuelle Anpassung ermöglicht ist.

Da die Ansatzplatten separat von der Hülse gefertigt werden können, besteht auch die Möglichkeit, diesen in Längsrichtung der Hülse weisenden Dichtegradienten zuzuordnen, um so eine Elastizität zu erreichen.

Eine weitere alternative Ausführungsform ist dadurch gekennzeichnet, dass die Ansatzplatten zur Anlage an die benachbarten Wirbelkörper bestimmte Stifte tragen, die in einem flexiblen Polster gelagert sind, so dass auf diese Weise eine gleichmäßge Anlage an die Wirbelkörperendplatten ermöglicht ist.

Eine Berücksichtigung der natürlichen Krümmung der Wirbelsäule wird ermöglicht, indem die Endplatten gelenkig an der Hülse gelagert sind.

Ganz besonders bevorzugt im Rahmen der Erfindung ist es, wenn das Implantteil mehrteilig mit mindestens einem ersten Implantatteil und einem zweiten Implantatteil gebildet ist, die in Richtung ihrer Längsache relativ zueinander verstellbar sind, um so eine Distraktionsmöglichkeit zu schaffen, also das Implantat in einer kurzen Konfiguration einfach in die zwischen den beiden Wirbelkörpern bestehende Lücke eingeführt werden kann, um nachfolgend im Laufe der Operation auf die erforderliche Länge distrahiert zu werden. Die Verstellbarkeit in Richtung der Längsachse läßt sich dabei in besonders einfacher Weise erzielen, indem das erste Implantatteil und das zweite Implantatteil durch ein Distraktionsgewinde miteinander verbunden sind, wobei wiederum zur Erweiterung der konkreten Realisierung des Distraktionsgewindes auf die eingangs genannten Druckschriften derselben Anmelderin verwiesen werden können.

Im folgenden wird die Erfindung an in der Zeichnung dargestellten Ausführungsbeispielen näher erläutert; es zeigen:

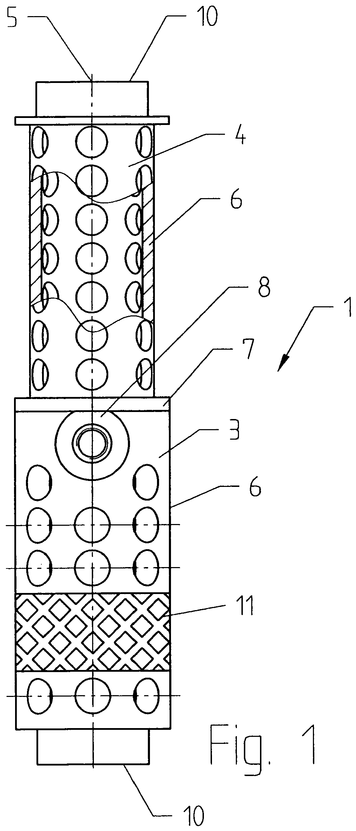

- Fig. 1: eine Seitenansicht eines erfindungsgemäßen Implantats, teilweise geschnitten dargestellt mit einem in der Wandung der Hülse in einer Wabenstruktur ausgebildeten elastischen nachgiebigen Bauteil,

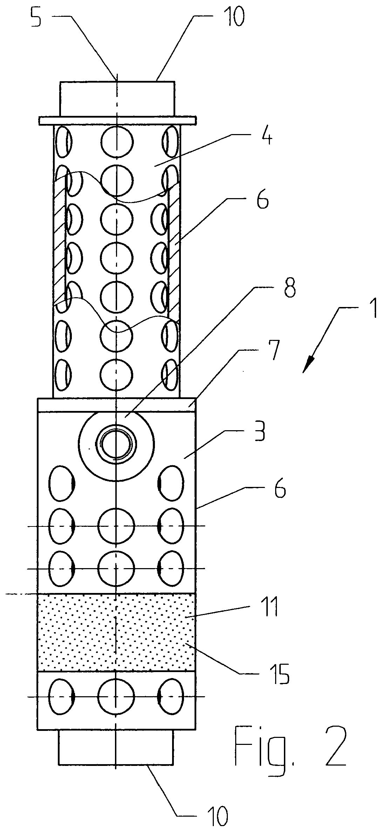

- Fig. 2: eine der Figur 1 entsprechende Darstellung einer weiteren Ausführungsform mit einem in die Wandung der Hülse integrierten elastisch nachgiebigen Bauteil aus einem elastisch kompakten Material,

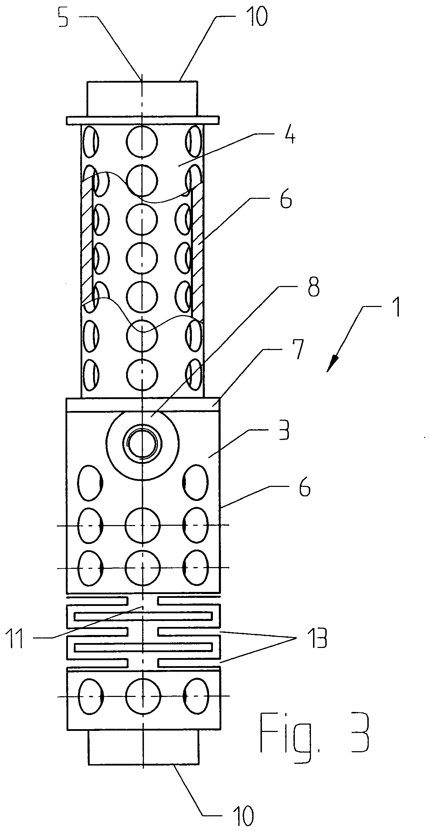

- Fig. 3: eine der Figur 1 entsprechende Darstellung einer Ausführungsform mit in der Wandung der Hülse eine kreuzweise versetzter Anordnung ausgebildeten Schlitzen,

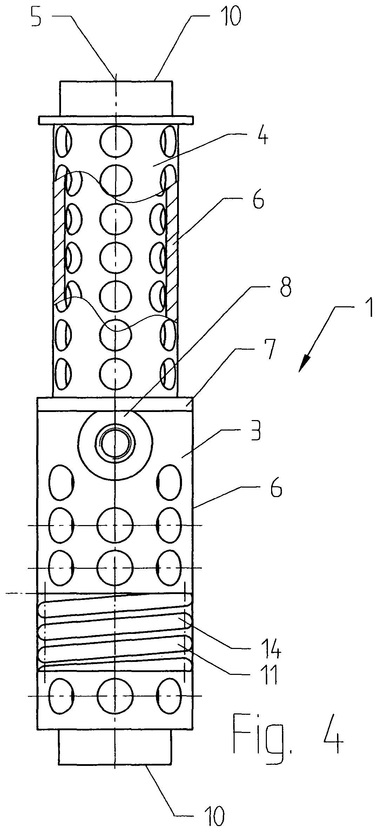

- Fig. 4: eine der Figur 1 entsprechende Darstellung einer Ausführungsform mit einer in der Wandung der Hülse integrierten Spiralfeder,

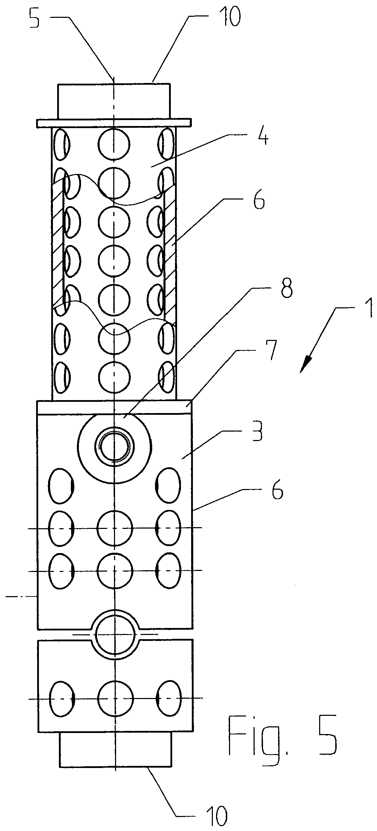

- Fig. 5: eine der Figur 1 entsprechende Darstellung einer Ausführungsform mit einem in der Wandung der Hülse integrierten, eine Rückstellfeder umfassenden Gelenk,

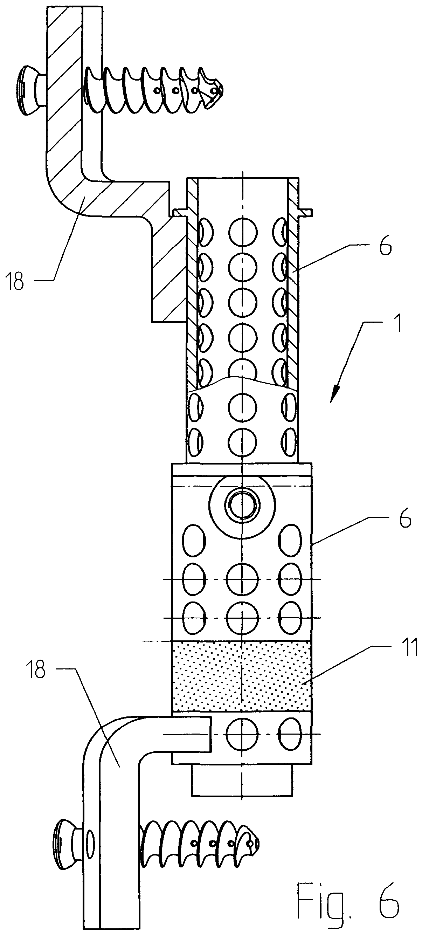

- Fig. 6: eine Darstellung der Ausführungsform aus Figur 2, verbunden mit dem Umgreifen der Wirbelkörper dienenden Laschen 18,

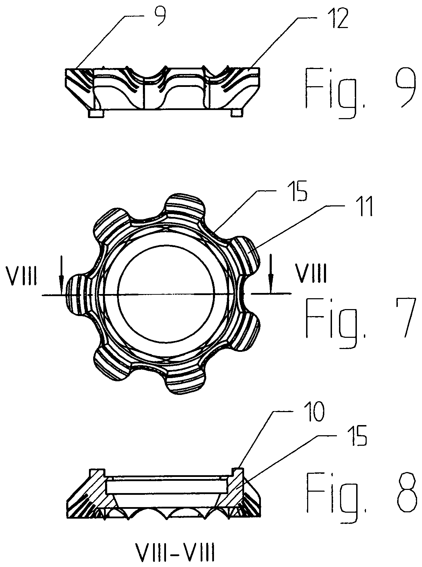

- Fig. 7: eine Draufsicht auf eine Ansatzplatte mit in ihrer Oberfläche integrierten Lamellen,
- Fig. 8: den Schnitt VIII-VIII aus Figur 7,
- Fig. 9: eine Seitenansicht der Ansatzplatte aus Figur 7,

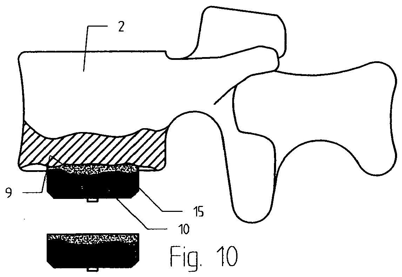

- Fig. 10: eine schematische Darstellung einer Ansatzplatte mit einem in Längsrichtung der Hülse weisenden Dichtegradienten im Kontakt mit der Anlagefläche des Wirbelkörpers,

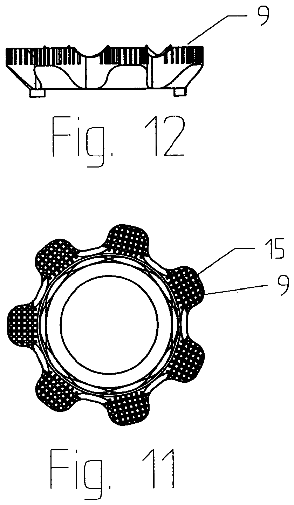

- Fig. 11: eine der Figur 7 entsprechende Darstellung einer weiteren Ansatzplatte,
- Fig. 12: eine Seitenansicht der Ansatzplatte aus Figur 11,

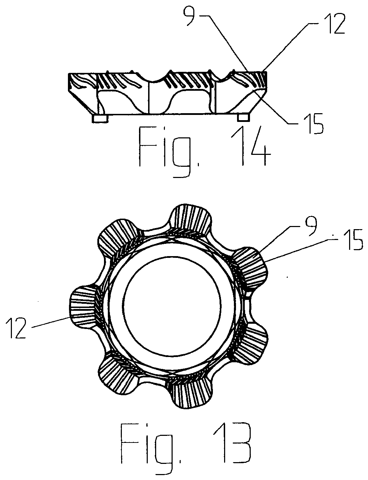

- Fig. 13: eine weitere Ausführungsform einer Ansatzplatte in einer der Figur 7 entsprechenden Darstellung,
- Fig. 14: eine Seitenansicht der Ansatzplatte aus Figur 13,

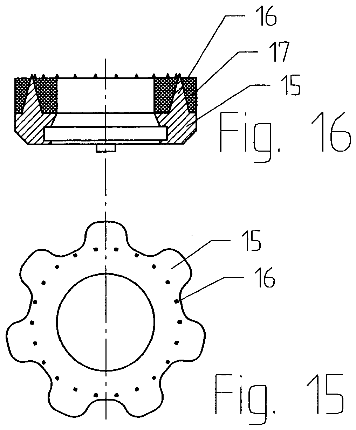

- Fig. 15: eine weitere Ausführungsform einer Ansatzplatte mit in einem flexiblen Polster gelagerten Stiften,
- Fig. 16: einen Längsschnitt durch die Ansatzplatte aus Figur 15,

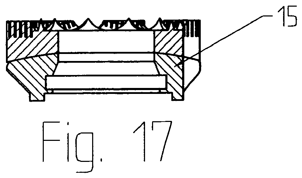

- Fig. 17: einen Längsschnitt durch eine zweiteilig aufgebaute Ansatzplatte, bestehend aus einer Oberschale sowie einer Unterschale, wobei die Oberschale eine Lamellenstruktur aufweist, und

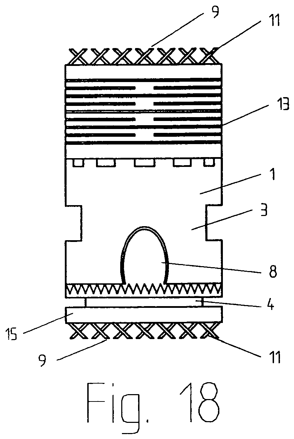

- Fig. 18: eine Seitenansicht eines erfindungsgemäßen Implantats mit in der Wandung der Hülse in kreuzweise versetzter Anordnung ausgebildeten Schlitzen sowie mit an der ersten Kontaktfläche und der zweiten Kontaktfläche angeordneten elastisch nachgiebigen Bauteilen.

Das in der Zeichnung dargestellte Implantat 1 dient zum Einsatz zwischen in der Zeichnung selber nur in Figur 10 dargestellten Wirbelkörpern 2 der Wirbelsäule. Dieses Implantat 1 weist ein erstes Implantatteil 3 und ein zweites Implantatteil 4 auf, die in Richtung ihrer koaxialen Längsachse 5 gegeneinander verstellbar sind, um im Sinne einer Distraktion der Wirbelkörper 2 eine Längenänderung bewirken zu können. Das erste Implantatteil 3 und das zweite Implantatteil 4 sind als rohrförmige Hülsen 6 gestaltet, wobei das erste Implantatteil 3 das zweite Implantatteil 4 außenseitig umfasst. Dem ersten Implantatteil 3 ist ein drehbarer Gewindering 7 zugeordnet, der an seiner Innenumfangsfläche eine Ringgewinde aufweist, mit dem dieser in einem dem zweiten Implantatteil 4 zugeordnetes Gewinde eingreift. An der Außenumfangsfläche ist der Gewindering 7 bei 8 mit einer Kegel- oder Kronenradverzahnung versehen, um so mittels eines in die Verzahnung eingreifenden Operationsinstrumentes die Verdrehung und damit die Längenänderung bewirken zu können.

Alternativ besteht auch die Möglichkeit, dass das Implantat 1 aus insgesamt zwei endständigen und einem mittigen Implantatteil besteht, wobei das mittige Implantatteil mit den endständigen Implantatteil über Gewindeverbindungen verbunden ist, die einen gegenläufigen Drehsinn aufweisen. Obwohl in der der Zeichnung zur Erläuterung der Ausführungsbeispiele stets Implantate 1 aus mindestens zwei Implantatteilen 3, 4 gezeigt sind, ist prinzipiell die Realisierung der Erfindung auch an einem Implantat 1 mit nur einem Implantatteil möglich, dem dann allerdings die Distraktionsmöglichkeit fehlt.

Dieses einstückige oder mehrteilige Implantatteil 3, 4 weist eine erste Kontakfläche 9 zur Anlage an einen der benachbarten Wirbelkörper 2 und eine zweite Kontaktfläche 10 zur Anlage an dem anderen benachbarten Wirbelkörper auf, wobei in der durch das Implantatteil 3, 4 gebildeten Kraftübertragungskette zwischen den Anlageflächen der benachbarten Wirbelkörper 2 mindestens ein elastisch nachgiebiges Bauteil 11 angeordnet ist, das bei dem in der Figur 18 dargestellten Ausführungsbeispiel mehrfach vorgesehen ist mit einer Zuordnung zu der ersten Kontaktfläche 9 sowie der zweiten Kontaktfläche 10.

Dabei besteht die Möglichkeit, dass das elastisch nachgiebige Bauteil 11 aus einem elastisch kompakten Material 15 gebildet ist, in dem Lamellen 12 zur Verfügung stehen. Eine weitere Möglichkeit besteht darin, dass das elastisch nachgiebige Bauteil 11 in einer elastischen Konfiguration bereitgestellt ist (Figur 18). Figur 18 läßt weiterhin erkennen, dass ergänzend in der Wandung der rohrförmigen Hülse 6 ein elastisch nachgiebiges Bauteil 11 integriert ist, wobei Figur 18 eine Anordnung zeigt mit Schlitzen 13, die in einer kreuzweise versetzten Anordnung realisiert sind, und zwar der Gestalt, dass in einer Radialebene der Hülse 6 zwei diametral gegenüberliegende Schlitze 13 ausgebildet sind, die gegenüber den Schlitzen 13 in den benachbarten Radialebene um 90° um die Längsachse gedreht angeordnet sind. Als Alternative zeigt die Figur 1 eine Ausbildung einer Wabenstruktur in der Wandung der Hülse 6. Figur 4 zeigt ein Ausführungsbeispiel mit einer integrierten Spiralfeder 14.

Weiterhin ist darauf hinzuweisen, dass den Hülsen 6 endständige Ansatzplatten 15 zugeordnet werden können, die die erste Kontaktfläche 9 und die zweite Kontaktfläche 10 bilden, wobei die Ansatzplatten 15 auch zur Bildung des elastisch nachgiebigen Bauteils 11 geeignet sind, dessen Realisierung beispielsweise durch einen in Längsrichtung der Hülse 6 weisenden Dichtegradienten möglich ist (Figur 10).

Die Figuren 15 und 16 zeigen Ausführungsformen, bei denen die Ansatzplatten 15 zur Anlage an die benachbarten Wirbelkörper 2 bestimmte Stifte 16 tragen, die in einem flexiblen Polster 17 gelagert sind.

## Patentansprüche

1. Implantat zum Einsetzen zwischen Wirbelkörper (2) der Wirbelsäule, das eine erste Kontaktfläche (9) zur Anlage an einen der benachbarten Wirbelkörper (2) und eine zweite Kontaktfläche (10) zur Anlage an den anderen benachbarten Wirbelkörper (2) aufweist und eine Kraftübertragungskette zwischen den Anlageflächen der benachbarten Wirbelkörper (2) bildet, in der mindestens ein elastisch nachgiebiges Bauteil (11) angeordnet ist, wobei das Implantat (1) mehrteilig mit mindestens einem ersten Impantatteil (3) und einem zweiten Implantatteil (4) gebildet ist, die in Richtung ihrer Längsachse (5) relativ zueinander verstellbar sind, wobei jedes der Implantatteile (3, 4) als rohrförmige Hülse (16) gebildet ist, **dadurch gekennzeichnet, dass** mindestens eines der Implantatteile (3, 4) das in der Wandung dieses Implantatteils integrierte elastische nachgiebige Bauteil (11) aufweist, das durch Schlitze (13) in einer kreuzweise versetzten Anordnung gebildet ist.

2. Implantat nach Anspruch 1, **dadurch gekennzeichnet, dass** das mindestens eine elastisch nachgiebige Bauteil (11) der ersten Kontaktfläche (9) und /oder der zweiten Kontaktfläche (16) zugeordnet ist.

3. Implantat nach Anspruch 1, **dadurch gekennzeichnet, dass** in einer Radialebene der Hülse (6) zwei diametral gegenüberliegende Schlitze (13) ausgebildet sind, die gegenüber den Schlitzen in den benachbarten Radialebenen um 90° um die Längsachse (5) der Hülse gedreht angeordnet sind.

4. Implantat nach Anspruch 1, **dadurch gekennzeichnet, dass** der Hülse (6) endständige Ansatzplatten (15) zugeordnet sind, die die erste Kontaktfläche (9) und die zweite Kontaktfläche (10) bilden.

5. Implantat nach Anspruch 4, **dadurch gekennzeichnet, dass** die Ansatzplatten (15) zur Anlage an die benachbarten Wirbelkörper (2) bestimmte Stifte (16) tragen, die in einem flexiblen Polster (17) gelagert sind.

6. Implantat nach Anspruch 5, **dadurch gekennzeichnet, dass** die Ansatzplatten gelenkig an der Hülse gelagert sind.

7. Implantat nach Anspruch 6, **dadurch gekennzeichnet, dass** das erste Implantatteil (3) und das zweite Implantatteil (4) durch ein Distraktionsgewinde miteinander verbunden sind.

## Claims

1. An implant for installation between vertebrae (2) of the spinal column having a first contact surface (9) for bearing against one of the adjacent vertebrae (2) and a second contact surface (10) for bearing against the other adjacent vertebral body (2), providing a force-transmission chain between the bearing surface of the adjacent vertebrae (2) in which at least one elastically compressible part (11) is arranged whereby the implant (1) is formed in a multi-part manner with at least one first implant part (3) and a second implant part (4) that are shiftable with respect to each other along their longitudinal axis (5), whereby each of the implant parts (3, 4) is formed as a tubular sleeve (6) **characterized in that** at least one of the implant parts (3, 4) in of which the wall the elastically compressible part (11) is integrated, which is formed with slots (13) in a crosswise offset arrangements.

2. The implant according to claim 1, **characterized in that** the at least one elastically compressible part (11) is assigned to the first contact surface (9) and/or to the second contact surface (16).

3. The implant according to claim 1, **characterized in that** two diametrically opposite slots (13) are formed in a radial plane of the sleeve (6), the slots being offset by 90° to the longitudinal axis (5) of the sleeve with respect to the slots in the adjacent radial planes.

4. The implant according to claim 1, **characterized in that** end adapter plates (15) are assigned to the sleeve (6) and form the first contact surface (9) and the second contact surface (10).

5. The implant according to claim 4, **characterized in that** the adapter plates (15) carry pins (16) that are supported in a flexible pad (17), for bearing against the adjacent vertebrae (2).

6. The implant according to claim 5, **characterized in that** the adapter plates (15) are pivoted on the sleeve.

7. The implant according to claim 6, **characterized in that** the first implant part (3) and the second implant part (4) are connected to one another by a distraction screwthread.

## Revendications

1. Implant, destiné à être mis en place entre des corps vertébraux (2) du rachis, qui présente une première surface de contact (9), pour une application contre l'un des corps vertébraux (2) adjacents, et une deuxième surface de contact (10), pour une application contre l'autre corps vertébral (2) adjacent, et forme une chaîne de transmission de force entre les surfaces d'appui des corps vertébraux (2) adjacents, dans laquelle est disposé au moins un composant (11) souple élastique, l'implant (1) étant réalisé en plusieurs parties, avec au moins une première partie d'implant (3) et une deuxième partie d'implant (4) qui peuvent être déplacées l'une par rapport à l'autre dans le sens de leur axe longitudinal (5), chacune des parties d'implant (3, 4) étant réalisée sous forme de manchon (16) tubulaire, **caractérisé en ce qu'**au moins une des parties d'implant (3, 4) présente le composant (11) souple élastique qui est intégré dans la paroi de cette partie d'implant et est formé par des fentes (13) dans une configuration décalée en forme de croix.

2. Implant selon la revendication 1, **caractérisé en ce que** le composant (11) souple élastique, au nombre d'au moins un, est associé à la première surface de contact (9) et/ou à la deuxième surface de contact (16).

3. Implant selon la revendication 1, **caractérisé en ce que**, dans un plan radial du manchon (6), il est prévu deux fentes (13) diamétralement opposées qui sont disposées en étant tournées de 90° autour de l'axe longitudinal (5) du manchon (6), par rapport aux fentes dans les plans radiaux adjacents.

4. Implant selon la revendication 1, **caractérisé en ce que**, sont associées au manchon (6), des plaques à épaulements (15) qui sont situées aux extrémités et constituent la première surface de contact (9) et la deuxième surface de contact (10).

5. Implant selon la revendication 4, **caractérisé en ce que** les plaques à épaulements (15) portent des picots (16) qui sont destinés à venir en appui contre les corps vertébraux (2) adjacents et sont fixés dans un rembourrage (17) flexible.

6. Implant selon la revendication 5, **caractérisé en ce que** les plaques à épaulements (15) sont montées de façon articulée sur le manchon.

7. Implant selon la revendication 6, **caractérisé en ce que** la première partie d'implant (3) et la deuxième partie d'implant (4) sont reliées entre elles par un filetage de distraction.
